# FASCICULE DE BREVET EUROPEEN

(11) **EP 2 588 638 B1**
(45) Date de publication et mention de la délivrance du brevet: **15.03.2017**
(21) Numéro de dépôt: 11741623.0
(22) Date de dépôt: 01.07.2011
(51) Int. Cl.: C23C 14/48, A61F 9/00, A61L 2/16, C08J 7/18, H01J 37/317, A61J 1/00, A61M 15/00, B65D 1/00, B05B 11/00, A61M 11/00

(54) **PROCEDE DE TRAITEMENT DE SURFACE ELASTOMERE D'UN DISPOSITIF DE DISTRIBUTION DE PRODUIT FLUIDE.**
VERFAHREN ZUR BEHANDLUNG EINER ELASTOMEROBERFLÄCHE EINER VORRICHTUNG ZUR AUSGABE EINES FLÜSSIGPRODUKTS
METHOD FOR TREATING AN ELASTOMERIC SURFACE OF A DEVICE FOR DISPENSING A FLUID PRODUCT

(30) Priorité: 08.07.2010 FR 1002868; 02.07.2010 FR 1055358
(43) Date de publication de la demande: 08.05.2013
(73) Titulaire: Aptar France SAS, 27110 Le Neubourg (FR)
(72) Inventeur: LEGOGUELIN, Marie, F-76100 Rouen (FR); LÉONÉ, Patrice, F-27400 Acquigny (FR); BUSARDO, Denis, F-14510 Gonneville Sur Mer (FR); GUERNALEC, Frédéric, F-35340 Liffre (FR)
(74) Mandataire: CAPRI
(86) Numéro de dépôt international: PCT/FR2011/051540
(87) Numéro de publication internationale: WO 2012/001321

(56) Documents cités:
- WO-A1-2010/037914
- WO-A1-2010/100384
- WO-A2-2007/115309
- WO-A2-2008/141141
- GB-A- 2 071 673
- US-A- 5 223 309
- SIOSHANSI P ET AL: "SURFACE TREATMENT OF BIOMATERIALS BY ION BEAM PROCESSES", SURFACE AND COATINGS TECHNOLOGY, ELSEVIER, AMSTERDAM, NL, vol. 83, no. 1/03, 1 janvier 1996 (1996-01-01), pages 175-182, XP000937529, ISSN: 0257-8972, DOI: DOI:10.1016/0257-8972(95)02838-2

## Description

La présente invention concerne un procédé de traitement de surface élastomère pour des dispositifs de distribution de produits fluides.

Les dispositifs de distribution de produits fluides sont bien connus. Ils comportent généralement un réservoir, un organe de distribution, tel qu'une pompe ou une valve, et une tête de distribution pourvue d'un orifice de distribution. Les pièces en élastomère, tels que les joints, présentent certains inconvénients, en particulier lors des phases de fabrication et d'assemblage. Ainsi, pour éviter des collages susceptibles de bloquer la chaîne de fabrication et/ou d'assemblage, les joints doivent être talqués, lavés et séchés. Ces processus compliquent la fabrication et l'assemblage des dispositifs de distribution concernés. Des problèmes similaires peuvent se poser avec d'autres pièces en élastomère, par exemple les pistons de pompe.

La présente invention a pour but de proposer un procédé de traitement de surface élastomère, notamment de joint, qui évite les inconvénients susmentionnés.

En particulier, la présente invention a pour but de fournir un procédé de traitement de surface élastomère qui soit efficace, durable, non polluant et simple à réaliser.

L'invention a en particulier pour objet un procédé de traitement d'une pièce en polymère élastomère par des ions multichargés et multi-énergies appartenant à la liste constituées de l'hélium (He), de l'azote (N), de l'oxygène (O), du néon (Ne), de l'argon (Ar), du krypton (Kr), du xénon (Xe), cette pièce en polymère faisant partie d'un dispositif de distribution de produit fluide, notamment pharmaceutique.

La plupart des polymères commercialisés ne conduisent pas le courant électrique. Leur résistivité superficielle se situe entre 10¹⁵ et 10¹⁷ Ω/□.

Mais la conduction électrique peut être recherchée pour plusieurs raisons, parmi lesquelles:
- l'effet antistatique : un abaissement de la résistivité superficielle pendant quelques semaines ou quelques mois peut être suffisant.
- la dissipation de charges électrostatiques : elle est obtenue avec des matériaux dissipatifs et des conducteurs qui préviennent des décharges électriques et dissipent les charges résultant des mouvements à grande vitesse.
- le blindage électromagnétique : il faut des matériaux avec une très faible résistivité volumique (<1 ohm.cm). Des normes sont à respecter pour limiter les émissions électromagnétiques des produits fabriqués.

La conductivité peut être obtenue par différentes voies :
- les additifs non permanents comme les esters d'amines gras ou les amines quaternaires. Ces substances, incorporées dans une matrice polymère, migrent à la surface et réagissent avec l'humidité de l'air. En formant un film humide à la surface, ils diminuent la résistivité superficielle à environ 10¹⁴ Ω/□.
- les charges qui abaissent la résistivité superficielle et volumique de façon permanente. Il s'agit notamment de noirs de carbone, de fibres de carbone, de graphite, de fibres d'inox, de paillettes d'aluminium, de nanotubes de carbone. Ces charges surenchérissent le coût d'élaboration du polymère lorsqu'on ne cherche que des propriétés électriques superficielles antistatiques, ou dissipatrices de charges électrostatiques.
- les polymères intrinsèquement conducteurs. Ces derniers sont à la fois chers et sensibles aux conditions d'utilisation. La chaleur et l'humidité ont vite fait de dégrader ses propriétés électriques.

L'adhésion est un phénomène important dans le cas des polymères qui se traduit par exemple par le collage de produit actif sur une surface. Cette adhésion résulte de la contribution des forces de Van Der Walls produites par la polarité des molécules situées à la surface du polymère et par les forces électrostatiques induites par la très haute résistivité superficielle.

Outre les problèmes de collage, les pièces en polymère doivent souvent fonctionner dans des milieux chimiques plus ou moins agressifs, à l'humidité ambiante, à l'oxygène ambiant etc., ce qui peut entraîner par oxydation un accroissement de leur caractère isolant électrique.

Certains polymères sont chargés en agents chimiques de protection contre les UV, l'oxydation. Le rejet de ces agents chimiques vers l'extérieur a pour effet d'accélérer l'oxydation superficielle qui renforce à son tour le caractère isolant du polymère.

L'invention a pour but de diminuer les inconvénients précités et notamment de permettre une forte diminution de la résistivité superficielle d'une pièce en polymère élastomère massive tout en conservant dans la masse ses propriétés élastiques et en évitant l'utilisation d'agents chimiques préjudiciables à la santé.

L'invention a donc notamment pour objet un procédé de traitement d'au moins une surface d'une pièce massive en polymère élastomère par des ions d'hélium caractérisée en ce qu'on implante simultanément des ions multi-énergies X⁺ et X²⁺, où le ratio RX, où RX = X⁺/X²⁺ ou X appartient à la liste constituée de l'hélium (He), l'azote (N), l'oxygène (O), le néon (Ne), l'argon (Ar), le krypton (Kr), le xénon (Xe) avec X⁺ et X²⁺ exprimés en pourcentage atomique est inférieur ou égal à 100, par exemple inférieur à 20.

Les inventeurs ont pu par exemple constater que la présence simultanée d'ions He⁺ et He²⁺ permet d'améliorer très significativement des propriétés antistatiques de surface des polymères élastomère par rapport aux traitements connus où seuls des ions de He⁺ ou d'He²⁺ sont implantés. Ils ont pu démontrer qu'une amélioration significative était obtenue pour RHe inférieur ou égal à 100, par exemple inférieur ou égal à 20.

On note que l'invention permet de réduire la résistivité superficielle d'une pièce en polymère élastomère massif, et/ou de supprimer le collage de la poussière ou autre, voire de réduire la polarisation de la surface en supprimant des groupes chimiques fortement polarisées comme OH, COOH. Ces groupes fonctionnels peuvent induire des forces de Van der Walls qui ont pour effet de coller des molécules chimiques ambiantes à la surface du polymère.

L'invention permet par ailleurs d'augmenter la stabilité chimique du polymère en créant par exemple une barrière de perméation. Cette dernière peut ralentir la propagation de l'oxygène ambiant dans le polymère, et/ou retarder la diffusion d'agents de protection chimiques contenus dans le polymère vers l'extérieur, et/ou inhiber le relargage d'agents toxiques contenu dans le polymère vers l'extérieur.

Avantageusement, elle permet de supprimer l'ajout d'agents chimiques ou de charges et de leur substituer un procédé physique applicable à tout type de polymère qui est peu coûteux en consommation de matière et d'énergie.

Dans le cadre de la présente invention, on entend par « massive » une pièce en polymère produite par transformation mécanique ou physique d'un bloc de matière, par exemple par extrusion, moulage ou toute autre technique adaptée à transformer un bloc polymère.

Grâce au procédé de la présente invention, on peut traiter des profondeurs beaucoup plus importante présentant une grande stabilité chimique se traduisant par une très longue conservation des propriétés électriques superficielles (antistatiques, dissipatrices de charges électrostatiques).

On constate que les temps de traitement ne sont pas longs au regard des exigences industrielles.

En outre, le procédé est peu énergétique, peu coûteux et permet son utilisation dans un cadre industriel sans aucun impact environnemental.

Le traitement d'une pièce en polymère élastomère est effectué par implantation simultanée d'ions multichargés multi-énergies. Ces derniers sont notamment obtenus en extrayant avec une même et unique tension d'extraction des ions mono- et multi-chargés créés dans la chambre à plasma d'une source d'ions à résonance cyclotronique électronique (source RCE). Chaque ion produit par ladite source présente une énergie qui est proportionnelle à son état de charge. Il en découle que les ions dont l'état de charge est le plus élevé, donc d'énergie la plus élevée, s'implantent dans la pièce en polymères à des profondeurs plus importantes.

Une implantation avec une source RCE est rapide et peu coûteuse puisqu'elle ne nécessite pas une tension d'extraction élevée de la source d'ions. En effet, pour augmenter l'énergie d'implantation d'un ion, il est économiquement préférable d'augmenter son état de charge plutôt que d'augmenter sa tension d'extraction.

Il convient de noter que l'utilisation d'une source classique, comme notamment les sources permettant l'implantation d'ions par immersion plasma ou les implanteurs à filament, ne permet pas d'obtenir un faisceau adapté à l'implantation simultanée d'ions multi-énergies X⁺ et X²⁺, où le ratio RX est inférieur ou égal à 100. Avec de telles sources, il est au contraire généralement supérieur ou égal à 1000.

Les inventeurs ont pu constater que ce procédé permet de traiter superficiellement une pièce en polymère élastomère sans altérer les propriétés élastiques de masse.

Selon un mode de réalisation de la présente invention, la source est une source à résonance cyclotronique électronique produisant des ions multi-énergies qui sont implantés dans la pièce à une température inférieure à 50°C et l'implantation des ions du faisceau d'implantation est effectuée simultanément à une profondeur contrôlée par la tension d'extraction de la source.

Sans vouloir être lié par une quelconque théorie scientifique, on peut penser que dans le procédé, selon l'invention, les ions excitent lors de leur passage les électrons du polymère provoquant des scissions de liaisons covalentes qui se recombinent aussitôt pour engendrer, par un mécanisme dit de réticulation, une haute densité de liaisons chimiques covalentes majoritairement constituées de carbones. Les éléments plus légers comme l'hydrogène et l'oxygène sont évacués du polymère lors d'un dégazage. Cette densification en liaisons covalentes riches en carbone a pour effet d'augmenter superficiellement conductivité et de réduire voire de supprimer les groupements polaires superficiels à l'origine des forces de Van der Walls à l'origine du collage. Le processus de réticulation est d'autant plus efficace que l'ion est léger.

L'hélium est à ce titre un projectile avantageux que l'on favorisera puisque :
- il est très rapide au regard de la vitesse des électrons des liaisons de covalence donc très efficace pour exciter ces mêmes électrons qui n'ont pas le temps de modifier en conséquence leurs orbitales,
- il pénètre des profondeurs importantes, de l'ordre du micron,
- il n'est pas dangereux,
- il n'intervient pas en tant que gaz noble dans la composition chimique du polymère.

D'autres types d'ions faciles dans la mise en oeuvre sans risque pour la santé sont envisageables comme l'azote (N), l'oxygène (O), le néon (Ne), l'argon (Ar), le krypton (Kr), le xénon (Xe).

Selon différents modes préférentiels de réalisation du procédé de la présente invention, qui peuvent être combinés entre eux. Un mode préféré consiste par exemple à combiner:
- le ratio RHe, où RHe = He⁺/He²⁺ avec He⁺ et He²⁺ exprimés en pourcentage atomique, est supérieur ou égal à 1;
- la tension d'extraction de la source permettant l'implantation des ions multi-énergies He⁺ et He²⁺ est comprise entre 10 et 400 kV, par exemple supérieure ou égale à 20 kV et/ou inférieure ou égale à 100 kV ;
- la dose d'ion multi-énergie He⁺ et He²⁺ est comprise entre 5.10¹⁴ et 10¹⁸ ions/cm², par exemple supérieure ou égale à 10¹⁵ ions/cm² et/ou inférieure ou égale à 5.10¹⁷ ions/cm², voire supérieure ou égale à 5.10¹⁵ ions/cm² et/ou inférieure ou égale à 10¹⁷ ions/cm² ;
- on détermine dans une étape préalable la variation d'une propriété caractéristique de l'évolution de la surface d'une pièce massive en polymère, par exemple de la résistivité superficielle du polymère d'un matériau polymère représentatif de celui de la pièce à traiter en fonction de doses d'ions multi-énergie He⁺ et He²⁺ de manière à déterminer un domaine de doses d'ions où la variation de la propriété caractéristique choisie est avantageuse et évolue de manière différenciée dans trois zones consécutives de doses d'ions formant ledit domaine de doses d'ions, avec une évolution dans la première zone sensiblement linéaire et réversible sur une durée inférieure à un mois, une évolution dans la deuxième zone sensiblement linéaire et stable sur une durée supérieure à un mois enfin une évolution dans la troisième zone constante et stable sur une durée supérieure à un mois et où l'on choisit la dose d'ions multi énergie He⁺ et He²⁺ dans la troisième zone de doses d'ions pour traiter la pièce massive en polymère; on entend par évolution réversible (première zone) le fait que la résistivité diminue puis remonte pour retrouver sa valeur d'origine. Ce phénomène est dû à la persistance de radicaux libres après implantation qui se recombinent avec l'oxygène de l'air ambiant provoquant ainsi une augmentation de la résistivité superficielle.
- on règle des paramètres de la source et de déplacement de la surface de la pièce en polymère à traiter de manière à ce que la vitesse surfacique de traitement de la surface de la pièce en polymère à traiter soit comprise entre 0,5 cm²/s et 1000 cm²/s, par exemple supérieure ou égale à 1 cm²/s et/ou inférieure ou égale à 100 cm²/s ;
- on règle des paramètres de la source et de déplacement de la surface de la pièce en polymère à traiter de manière à ce que la dose d'hélium implantée soit comprise entre 5.10¹⁴ et 10¹⁸ ions/cm², par exemple supérieure ou égale à 5.10¹⁵ ions/cm² et/ou inférieure ou égale à 10¹⁷ ions/cm² ;
- on règle des paramètres de la source et de déplacement de la surface de la pièce en polymère à traiter de manière à ce que la profondeur de pénétration de l'hélium sur la surface de la pièce en polymère traitée soit comprise entre 0,05 et 3 µm, par exemple supérieure ou égale à 0,1 µm et/ou inférieure ou égale à 2 µm ;
- on règle des paramètres de la source et de déplacement de la surface de la pièce en polymère à traiter de manière à ce que la température de la surface de la pièce en polymère en cours de traitement soit inférieure ou égale à 100°C, par exemple inférieure ou égale à 50°C ;
- la pièce à traiter est par exemple une bande profilée, et ladite pièce défile dans un dispositif de traitement, par exemple à une vitesse comprise entre 5 m/min et 100 m/min ; à titre d'exemple, la pièce à traiter est une bande profilée qui défile longitudinalement ;
- l'implantation d'hélium de la surface de la pièce à traiter est effectuée grâce à une pluralité de faisceaux d'ions multi énergies He⁺ et He²⁺ produits par une pluralité de sources d'ions ; à titre d'exemple, les sources d'ions sont disposées le long d'une direction de déplacement de la pièce à traiter ; de préférence les sources sont espacées de manière à ce que la distance entre deux faisceaux d'ions soit suffisante pour permettre à la pièce de se refroidir entre chaque implantation d'ions successive; lesdites sources produisent des faisceaux d'ions dont le diamètre est adapté à la largeur des pistes à traiter. En diminuant le diamètre des faisceaux à par exemple 5 mm on est capable de mettre un système de vide différentiel très efficace entre la source et la chambre de traitement, permettant de traiter les polymères à 10⁻² mbar alors que le vide au niveau du système d'extraction de la source est de 10⁻⁶ mbar ;

L'invention porte également sur une pièce où l'épaisseur où l'hélium est implanté est supérieure ou égale à 50 nm, par exemple supérieure ou égale à 200 nm et dont la résistivité superficielle p, est inférieure ou égale à 10¹⁴ Ω/□, par exemple inférieure ou égale à 10⁹ Ω/□, voire inférieure ou égale à 10⁵ Ω/□. Pour la mesure de résistivité superficielle on se reportera à la norme CEI 60093.

La présente invention a donc pour objet un procédé de traitement de surface élastomère d'un dispositif de distribution de produit fluide tel que décrit dans la revendication 1.

Des modes de réalisation avantageux sont décrits dans les revendications dépendantes.

En particulier, ledit procédé comprend de traiter au moins une surface d'une pièce massive en polymère élastomère par des ions, ledit procédé comprenant un bombardement ionique par un faisceau d'ion constitué d'ions multi-énergies X⁺ et X²⁺, où X est le symbole atomique de l'ion choisi parmi la liste comprenant l'hélium (He), l'azote (N), l'oxygène (O), le néon (Ne), l'argon (Ar), le krypton (Kr), le xénon (Xe), dans lequel RX = X⁺/X²⁺ avec X⁺ et X²⁺ exprimés en pourcentage atomique est inférieur ou égal à 100, par exemple inférieur à 20, dans lequel la vitesse de déplacement du faisceau est déterminée dans une étape préalable où l'on identifie la plus petite vitesse de déplacement du faisceau qui ne provoque pas une dégradation thermique du polymère se manifestant par une remontée de pression de 10⁻⁵ mbar.

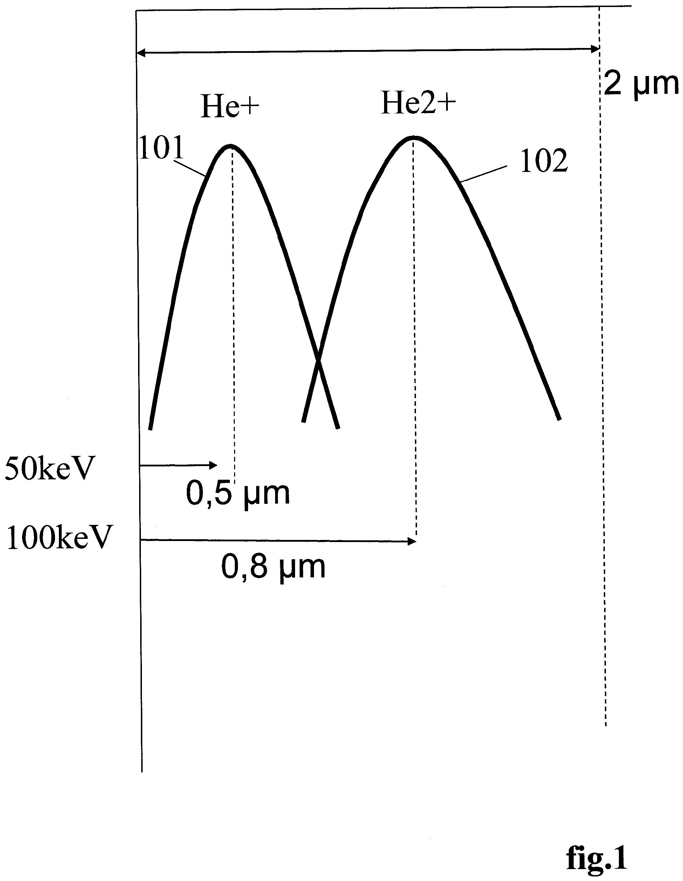

Ces caractéristiques et avantages et d'autres de la présente invention apparaîtront plus clairement dans la description détaillée ci-après, faite notamment en référence aux dessins joints, donnés à titre d'exemples non limitatifs, et sur lesquels
- la figure 1 représente un exemple de distribution d'implantation d'hélium selon l'invention dans un polycarbonate ;

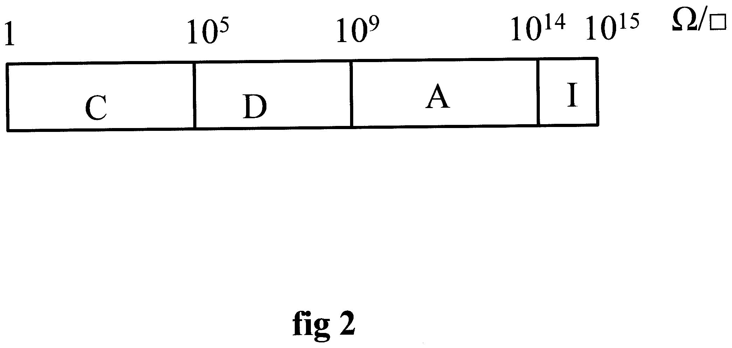

- la figure 2 représente les échelles selon différentes normes qualifiant les propriétés électrostatiques d'un matériau ;

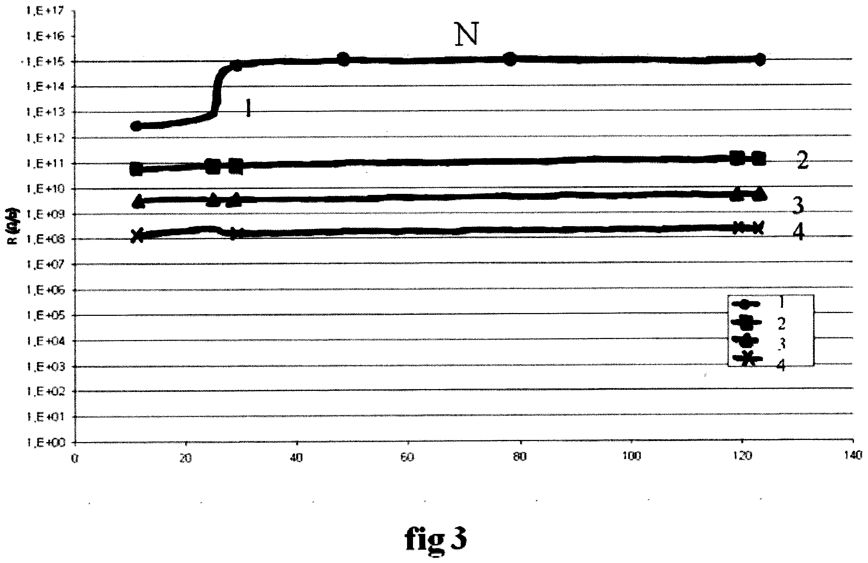

- la figure 3 représente la variation de la résistivité superficielle de la surface d'un échantillon polycarbonate traité selon l'invention, en fonction du temps, pour une pluralité de doses d'hélium ; la résistivité superficielle a été mesurée selon la norme CEI 60093, en mettant en oeuvre une électrode constituée d'un disque de diamètre d, entourée par une couronne de diamètre intérieur D où D est supérieur à d ;

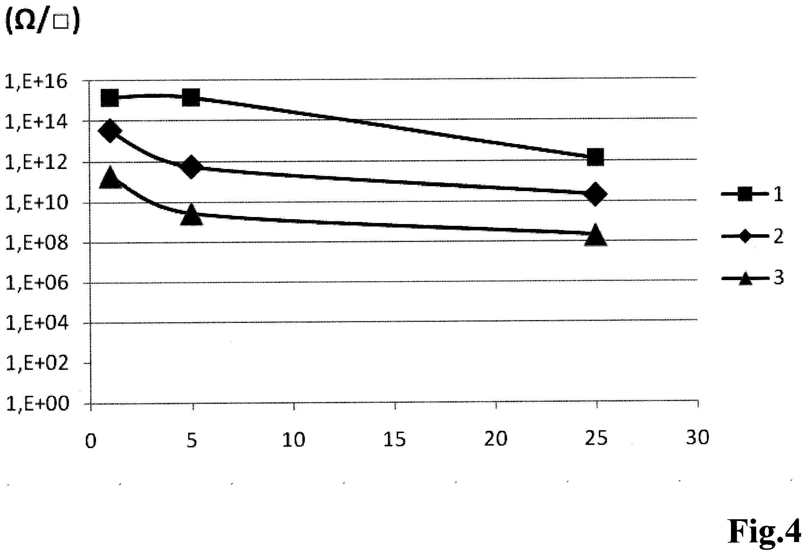

- la figure 4 représente la variation de la résistivité superficielle de la surface d'un échantillon polycarbonate traité selon l'invention, en fonction du temps, pour trois types d'ions He, N, Ar selon une pluralité de doses ; la résistivité superficielle a été mesurée selon la norme CEI 60093 ; et

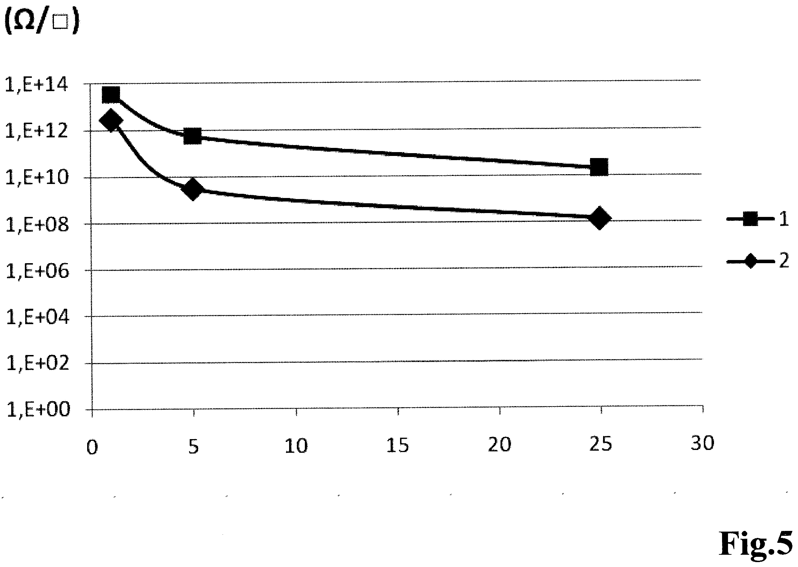

- la figure 5 représente la variation de la résistivité superficielle de la surface d'un échantillon polycarbonate traité selon l'invention, en fonction du temps, pour une pluralité de doses d'azote mais selon deux vitesses de déplacement faisceau ; la résistivité superficielle a été mesurée selon la norme CEI 60093.

En particulier, la présente invention prévoit d'utiliser un procédé similaire à celui décrit dans le document WO 2005/085491, qui concerne un procédé d'implantation ionique, et plus particulièrement l'utilisation d'un faisceau d'ions multichargés multi énergies afin de modifier structurellement la surface de matériaux métalliques sur des profondeurs autour du µm pour leur conférer des propriétés physiques particulières. Ce procédé d'implantation a notamment été utilisé pour traiter des pièces réalisées en alliage d'aluminium qui sont utilisées comme moules de fabrication en série de pièces en matière plastique.

De manière surprenante, ce type de procédé s'est avéré adapté pour éviter le collage de surfaces élastomères entre elles, notamment lors de la fabrication des joints de dispositifs de distribution de produit fluide. Une telle application de ce procédé d'implantation ionique n'avait jamais été envisagée. Elle évite les opérations de talcage habituellement nécessaires lors de la fabrication et l'assemblage des joints des dispositifs de distribution de produits fluides. La totalité de la description de ce document WO 2005/085491 est donc incorporé dans la présente description à titre de référence.

La surface élastomère est de préférence un joint de col ou un joint de soupape d'un dispositif de distribution de produit fluide pharmaceutique. Ce joint peut être en tout matériau élastomère approprié, tel que l'EPDM, le chloroprène, le nitrile, le HNBR, etc.

De manière simplifiée, le procédé consiste à utiliser une ou plusieurs sources d'ions, telles qu'une source à résonance cyclotronique électronique, dite source RCE. Cette source RCE peut délivrer un faisceau initial d'ions multi-énergies, par exemple ayant un courant total d'environ 10 mA (toutes charges confondues), sous une tension d'extraction pouvant varier de 20 kV à 200 kV. La source RCE émet le faisceau d'ions en direction de moyens de réglage qui assurent la focalisation et le réglage du faisceau initial émis par la source RCE en un faisceau d'implantation d'ions qui vient frapper une pièce à traiter. Selon les applications et les matériaux à traiter, les ions peuvent être choisis parmi l'hélium, le bore, le carbone, l'azote, l'oxygène, le néon, l'argon, le krypton et le xénon. De même, la température maximale de la pièce à traiter varie en fonction de sa nature. La profondeur d'implantation typique est entre 0 et 3 µm, et dépend non seulement de la surface à traiter mais aussi des propriétés qu'on souhaite améliorer.

La spécificité d'une source d'ions RCE réside notamment dans le fait qu'elle délivre des ions mono- et multichargés, ce qui permet d'implanter simultanément des ions multi-énergies avec la même tension d'extraction. Il est ainsi possible d'obtenir simultanément sur toute l'épaisseur traitée un profil d'implantation mieux réparti. Ceci améliore la qualité du traitement de surface.

Avantageusement, le procédé est effectué dans une enceinte mise sous vide grâce à une pompe à vide. Ce vide a pour but notamment d'empêcher l'interception du faisceau par des gaz résiduels et d'éviter la contamination de la surface de la pièce par ces mêmes gaz lors de l'implantation.

Avantageusement, comme notamment décrit dans le document WO 2005/085491, les moyens de réglage ci-dessus mentionnés peuvent comporter, de la source RCE vers la pièce à traiter, les éléments suivants:
- un spectromètre de masse apte à filtrer les ions en fonction de leur charge et de leur masse. Un tel spectromètre est toutefois facultatif si l'on injecte un gaz pur, par exemple un gaz d'azote pur (N2). Il est alors possible de récupérer l'ensemble des ions mono- et multichargés produits par la source pour obtenir un faisceau d'ions multi-énergies.
- une ou plusieurs lentilles pour donner au faisceau d'ions une forme prédéterminée, par exemple cylindrique, avec un rayon prédéterminé.
- un profileur pour analyser lors de la première implantation l'intensité du faisceau dans un plan de coupe perpendiculaire.
- un transformateur d'intensité pour mesurer en continu l'intensité du faisceau d'ions sans l'intercepter. Cet instrument a notamment pour fonction de détecter toute interruption du faisceau d'ions et de permettre l'enregistrement des variations d'intensité du faisceau durant le traitement.
- un obturateur, qui peut par exemple être une cage de Faraday, pour interrompre la trajectoire des ions à certains moments, par exemple lors d'un déplacement sans traitement de la pièce.

Selon une forme avantageuse de réalisation, la pièce à traiter est mobile par rapport à la source RCE. La pièce peut par exemple être montée sur un support mobile dont le déplacement peut être commandé par une machine à commande numérique. Le déplacement de la pièce à traiter est calculé en fonction du rayon du faisceau, des contours externes et internes des zones à traiter, de la vitesse de déplacement constante ou variable en fonction de l'angle du faisceau par rapport à la surface et du nombre de passes précédemment réalisées.

Une mise en oeuvre possible du procédé de traitement est la suivante. On fixe la pièce à traiter sur un support approprié dans une enceinte, puis on ferme l'enceinte et on y instaure un vide poussé au moyen d'une pompe à vide. Dès que les conditions de vide sont atteintes, on procède à la production et au réglage du faisceau d'ions. Lorsque ledit faisceau est réglé, on lève l'obturateur et on actionne la machine à commande numérique qui exécute alors le déplacement en position et en vitesse de la pièce à traiter devant le faisceau en une ou plusieurs passes. Lorsque le nombre de passes requis est atteint, on baisse l'obturateur pour couper le faisceau, on arrête la production du faisceau, on casse le vide en ouvrant l'enceinte à l'air ambiant, on arrête éventuellement le circuit de refroidissement et on sort la pièce traitée hors de l'enceinte.

Pour diminuer la température liée au passage du faisceau d'ions en un point donné de la pièce à traiter, on peut soit augmenter le rayon du faisceau (donc réduire la puissance par cm²), soit augmenter la vitesse de déplacement. Si la pièce est trop petite pour évacuer par rayonnement la chaleur liée au traitement, on peut soit diminuer la puissance du faisceau (donc augmenter la durée de traitement), soit mettre en marche le circuit de refroidissement.

Concernant plus particulièrement les élastomères, il est avantageux d'implanter simultanément des ions d'hélium multi-énergies He+ et He2+. Ceci a notamment été décrit dans le document PCT/FR2010/050379, qui est incorporé ici à titre de référence, et qui concerne plus particulièrement le traitement des balais d'essuie-glace pour véhicules. Avantageusement, le ratio RHe, où RHe = He+/He2+ avec He+ et He2+ exprimés en pourcentage atomique, est inférieur ou égal à 100, par exemple inférieur à 20, et de préférence supérieur à 1. Les ions He+ et He2+ sont avantageusement produits simultanément par une source RCE. La tension d'extraction de la source permettant l'implantation des ions multi-énergies He+ et He2+ peut être comprise entre 10 et 400 kV, par exemple supérieure ou égale à 20 kV et/ou inférieure ou égale à 100 kV. Avantageusement, la dose d'ion multi-énergie He+ et He2+ est comprise entre 1014 et 1018 ions/cm², par exemple supérieure ou égale à 1015 ions/cm² et/ou inférieure ou égale à 1017 ions/cm², voire supérieure ou égale à 1015 ions/cm² et/ou inférieure ou égale à 1016 ions/cm². La profondeur d'implantation est avantageusement entre 0,05 et 3 µm, par exemple entre 0,1 et 2 µm. La température de la surface élastomère en cours de traitement est avantageusement inférieure à 100°C, de préférence inférieure à 50°C.

Dans un mode de réalisation avantageux de l'invention, différentes implantations ioniques sont réalisées sur une même surface élastomère à traiter, pour donner plusieurs propriétés à cette surface élastomère à traiter. Ainsi, les surfaces élastomères, et notamment les joints susmentionnés sont susceptibles d'interagir avec le produit fluide, par exemple en relarguant des extractibles dans ledit produit fluide, ce qui peut avoir un effet néfaste sur celui-ci. L'invention prévoit avantageusement de modifier la surface élastomère par implantation ionique pour empêcher les interactions entre la surface élastomère et le produit fluide. On peut aussi envisager de modifier la surface élastomère par implantation ionique pour lui octroyer des propriétés anti-frottements, notamment pour faciliter les déplacements des pistons et soupapes dans les joints. D'autres traitements complémentaires sont aussi envisageables, pour notamment améliorer la résistance à l'oxydation, à l'usure et/ou à l'abrasion. Ces traitements de surfaces supplémentaires peuvent être appliqués lors d'étapes d'implantations ioniques successives. Il est à noter que l'ordre des ces étapes successives d'implantations ioniques peut être quelconque. En variante, différentes propriétés pourraient aussi être octroyées à une même surface à traiter lors d'une seule et même étape d'implantation ionique.

Le procédé de l'invention est non polluant, notamment du fait qu'il ne nécessite pas de produits chimiques. Il est réalisé à sec, ce qui évite les périodes de séchages relativement longues des procédés de traitement liquides. Il ne nécessite pas une atmosphère stérile en dehors de l'enceinte à vide, et il peut donc être réalisé en tout endroit souhaité. Un avantage particulier de ce procédé est qu'il peut être intégré dans la chaine de montage du dispositif de distribution de produit fluide, et opérer en continu dans cette chaine. Cette intégration du procédé de traitement à l'outil de production simplifie et accélère le processus de fabrication et d'assemblage dans son ensemble, et impacte donc positivement son coût.

Les figures 1 à 5 illustrent des modes de réalisation avantageux de l'invention.

La figure 1 représente un exemple schématique de distribution d'implantation de l'hélium en fonction de la profondeur selon l'invention, dans un polycarbonate. La description qui suit pourrait s'appliquer également aux polymères élastomères. La courbe 101 correspond à la distribution d'He⁺ et la courbe 102 à celle d'He²⁺. On peut estimer que pour des énergies de 100 keV, He²⁺ parcourt une distance moyenne d'environ 800 nm pour une énergie d'ionisation moyenne de 10 eV/Angstroem. Pour des énergies de 50 keV, He⁺ parcourt une distance moyenne d'environ 500 nm pour une énergie d'ionisation moyenne de 4 eV/Angstroem. L'énergie d'ionisation d'un ion est en rapport avec son pouvoir de réticulation. Dans le cas où (He⁺/He²⁺) est inférieur ou égal à 100, on peut estimer que l'épaisseur maximale traitée est de l'ordre de 1000 nm soit 1 micron. Ces estimations sont cohérentes avec des observations effectuées par microscopie électronique qui ont démontré que pour un faisceau extrait à 40 kV et une dose totale de 5.10¹⁵ ions/cm² et (He⁺/He²⁺) = 10, on observe une couche réticulée d'environ 750 à 850 nm.

La figure 2 représente selon la norme DOD HDBK 263, les valeurs de résistivité qualifiant les propriétés électrostatiques d'un matériau. Un polymère a des propriétés isolantes pour des valeurs de résistivité superficielle supérieures à 10¹⁴ Ω/□ (ZONE I), des propriétés antistatiques pour des valeurs de résistivité superficielle comprises entre 10¹⁴ Ω/□ et 10⁹ Ω/□ (zone A). Les propriétés dissipatrices de charges électrostatiques apparaissent pour des valeurs de résistivité superficielle comprises entre 10⁵ Ω/□ et 10⁹ Ω/□ (zone D) et des propriétés conductrices pour des valeurs inférieures à 10⁵ Ω/□ (zone C).

La figure 3 représente l'évolution expérimentale de la résistivité superficielle d'un polycarbonate en fonction du temps, pour différentes doses d'hélium égales à 10¹⁵ (courbe 1), 2,5.10¹⁵ (courbe 2), 5.10¹⁵ ions/cm² (courbe 3), 2,5.10¹⁶ ions/cm² (courbe 4) avec (He⁺/He²⁺) = 10, la tension d'extraction est d'environ 40 kV. La mesure de résistivité a été effectuée selon la norme CEI 60093. La technique de mesure de résistivité mise en oeuvre ne permet pas de mesurer des résistivités supérieures à 10¹⁵ Ω/□ correspondant à la zone N, elle sature à 10¹⁵ Ω/□. L'axe des abscisses correspond au temps séparant le traitement de l'échantillon à la mesure de sa résistivité superficielle. L'axe des ordonnées correspond à la mesure de la résistivité superficielle exprimée en Ω/□. On discerne une première zone pour les doses inférieures ou égales à 10¹⁵ ions/cm², la résistivité superficielle diminue pendant moins d'un mois d'environs 3 ordres de grandeur (passant 1,5.10¹⁶ Ω/□ à 5.10¹² Ω/□) avant de retrouver sa valeur d'origine située au alentour de 1,5.10¹⁶ Ω/□ (courbe 1). Dans cette zone les propriétés antistatiques sont éphémères, les radicaux libres encore présents se recombinent avec l'oxygène et l'air ambiant. Une deuxième zone ou l'on observe une évolution dégressive de la résistivité en fonction de la dose: entre 2,5.10¹⁵, 5.10¹⁵, 2,5.10¹⁶ ions/cm², la résistivité diminue passant de 10¹¹ Ω/□ à 5.10⁹ Ω/□ jusqu'à atteindre un plancher de saturation estimé aux alentours de 1,5.10⁸ Ω/□. Les propriétés antistatiques (courbe 2 et 3) se renforcent pour devenir dissipatrices de charges électrostatiques (courbe 4). Pour ces doses, les résistivités restent constantes sur plus de 140 jours. Pour des doses supérieures à 2,5.10¹⁶ ions/cm², on atteint une troisième zone ou l'évolution de la résistivité sature en fonction de la dose autour d'une valeur que l'on estime à 10⁸ Ω/□ et reste stable dans le temps sur plus de 140 jours.

La figure 4 représente l'évolution expérimentale de la résistivité superficielle d'un polycarbonate (PC) en fonction du temps, pour trois types d'ions He (courbe 1), N (courbe 2) et Ar (courbe 3) pour différentes doses égales à 10¹⁵, 5.10¹⁵ et 2,5.10¹⁶ ions/cm² avec (He⁺/He²⁺) = 10, (N⁺/N2⁺) = 2 et (Ar⁺/Ar²⁺) = 1,8. Le faisceau a un diamètre de 15 mm et une intensité de 0,225 mA, la tension d'extraction est d'environ 35 kV. L'axe des abscisses représente la dose en ions par unité de surface exprimée 10¹⁵ ions/cm². L'axe des ordonnées représente la résistivité superficielle exprimée en Ω/□. La mesure de résistivité a été effectuée selon la norme CEI 60093. Pour une même dose les ions les plus lourds sont les plus efficaces pour réduire la résistivité superficielle, le PC traité avec l'azote à une résistivité superficielle au moins 10 fois plus faible que celle du PC traité avec l'hélium, le PC traité avec de l'argon à une résistivité superficielle au moins 10 fois plus faible que celle du PC traité avec de l'azote et 100 fois plus faible que celle du PC traité avec de l'hélium. Les inventeurs préconisent l'emploi d'ions encore plus lourds comme le xénon pour réduire davantage la résistivité du polycarbonate.

La figure 5 représente l'évolution expérimentale de la résistivité superficielle d'un polycarbonate en fonction du temps, pour un même type d'ions mais deux vitesses de déplacement faisceau différentes, une vitesse de déplacement égale à 80 mm/s (courbe 1), une vitesse de déplacement égale à 40 mm/s (courbe 2) pour différentes doses égales à 10¹⁵, 5.10¹⁵ et 2,5.10¹⁶ ions/cm² (N⁺/N2⁺) = 2. Le faisceau a d'un diamètre de 15 mm et une intensité de 0,150 mA, la tension d'extraction est d'environ 35 kV. L'axe des abscisses représente la dose en ions par unité de surface exprimée 10¹⁵ ions/cm². L'axe des ordonnées représente la résistivité superficielle exprimée en Ω/□. La mesure de résistivité a été effectuée selon la norme CEI 60093. De ces courbes il ressort que la réduction de la vitesse d'un facteur 2 a pour effet de réduire d'un facteur 10 la résistivité superficielle du PC. Sans vouloir être lié par une quelconque théorie scientifique, on peut penser que qu'en réduisant la vitesse du faisceau on augmente la température superficielle du PC. Cette température favorise fortement la recombinaison des radicaux libres entre eux, favorisant du même coup la formation d'une couche dense et conductrice de carbone amorphe. L'échauffement à également pour effet d'expulser les gaz résiduels produits par les mécanismes de scission/réticulation induits par le bombardement ioniques. Les inventeurs ont déduit de cette expérience que pour tout polymère traité avec un faisceau d'un diamètre et d'une puissance connus, il existe une vitesse minimale de déplacement du faisceau provoquant une réduction maximale de la résistivité superficielle du polymère sans risque de dégradation du polymère sous l'effet de la chaleur produite. La dégradation thermique du polymère a pour signature un dégazage important suivie d'une remontée de pression au niveau du système d'extraction de la source RCE. Cette remontée de pression se traduit par des claquages électriques. Le système d'extraction sert à extraire les ions du plasma de la source RCE pour former le faisceau. Il est constituée de deux électrodes, la première est mise à la terre, la seconde est portée à une haute tension de plusieurs dizaines de kV (kilovolts) dans des conditions de vide inférieures à 5x10⁻⁶ mbar de préférence inférieures à 2x10⁻⁶ mbar. Au delà de ces pressions, des arcs électriques se produisent). C'est le cas lorsque la dégradation thermique du polymère survient. Il convient donc de détecter très tôt ces remontées de pressions en réduisant progressivement la vitesse de déplacement du faisceau et en suivant l'évolution de la pression au niveau du système d'extraction.

Pour déterminer cette vitesse de déplacement faisceau les inventeurs préconisent une étape d'essais qui consiste à réduire progressivement la vitesse du faisceau en conservant les autres caractéristiques:
- le caractéristique faisceau: diamètre, puissance autrement dit intensité et tension d'extraction
- les caractéristiques cinématiques : amplitude de déplacement, pas d'avancement.

Le polymère se dégrade thermiquement sous l'effet de la chaleur, lorsque la remontée de pression mesurée par une jauge située aussi bien au niveau du système d'extraction que dans la chambre de traitement, effectue un bond de 10⁻⁵ mbar en quelques secondes voire moins. Il faut arrêter immédiatement les essais pour ne conserver que la vitesse de déplacement du faisceau de l'essai précédent. Ce bond de 10⁻⁵ mbar en quelques seconde voire moins, constitue la signature d'une dégradation thermique du polymère.

Plusieurs méthodes de caractérisation ont permis de faire ressortir les avantages de la présente invention.

Dans les exemples qui suivent, le traitement d'au moins une surface d'une pièce en polymère massive par implantation d'ions d'hélium He⁺ et He²⁺ a été effectué avec des ions He⁺ et He²⁺ multi-énergies, produits simultanément par une source RCE. Les polymères traités ont notamment été les suivants : le polypropylène (PP), le polyméthylacrilate (PMMA).

Des essais comparatifs relatifs aux propriétés antistatiques avec des petits morceaux de papier jetés sur des échantillons traités ont permis de montrer que celle ci apparaissent pour des doses supérieures à 5.10¹⁵ ions/cm². Pour ces doses les morceaux de papier se détachent et tombent lorsque l'on retourne ces échantillons, ce qui n'est pas le cas pour des doses inférieures à 5.10¹⁵ ions/cm².

Pour le polypropylène, on a pu mesurer selon la norme CEI 60093 et pour des doses de 10¹⁵ et 5.10 ¹⁵ ions/cm², une résistivité de 10¹⁴ Ω/□. Pour une dose de 2.10 ¹⁶ ions/cm², on a pu mesurer une résistivité de 5.10¹¹ Ω/□ correspondant à l'apparition de ces propriétés antistatiques.

Selon un mode de réalisation, on estime que les propriétés antistatiques de surface d'un polymère sont significativement améliorées à partir d'une dose supérieure à 5.10¹⁵ ions/cm², ce qui représente une vitesse de traitement d'environ 15 cm²/s pour un faisceau d'Hélium constitué de 9 mA d'ion He⁺ et 1 mA d'ions He²⁺.

L'implantation simultanée des ions d'hélium peut se faire à des profondeurs variables, en fonction des besoins et de la forme de la pièce à traiter. Ces profondeurs dépendent notamment des énergies d'implantation des ions du faisceau d'implantation ; elles peuvent par exemple varier de 0,1 à environ 3 µm pour un polymère. Pour des applications ou l'on recherche plutôt des propriétés par exemple d'anti collage, on pourrait se contenter par exemple d'épaisseur inférieure au micron, réduisant d'autant les temps de traitement.

Selon un mode de réalisation, on choisit les conditions d'implantation des ions He⁺ et He²⁺ de manière à ce que la pièce en polymère conserve ses propriétés élastiques de masse grâce à un maintien de la pièce à des températures de traitement inférieures à 50°C. Ce résultat peut notamment être atteint pour un faisceau d'un diamètre de 4 mm délivrant une intensité totale de 60 microampères avec une tension d'extraction de 40 kV, se déplaçant à 40 mm/s sur des amplitudes de déplacement de 100 mm. Ce faisceau a une puissance par unité de surface de 20 W/cm². Pour utiliser avec la même tension d'extraction et la même puissance par unité de surface, des faisceaux d'intensité plus importante et conserver les propriétés élastiques de masse, on peut suggérer une règle d'échelle qui consiste à augmenter le diamètre du faisceau, à augmenter la vitesse de déplacement et à augmenter les amplitudes de déplacements, dans un rapport correspondant à la racine carrée (intensité désirée / 60 microampères). Par exemple pour une intensité de 6 milliampères (soit 100 fois 60 microampères), le faisceau peut avoir un diamètre de 40 mm pour conserver une puissance surfacique de 20 W/cm². Il convient dans ces conditions de multiplier la vitesse par un facteur 10 et les amplitudes de déplacement par un facteur 10, ce qui donne une vitesse de 40 cm/s et des amplitudes de déplacement de 1 m. Il convient également de multiplier le nombre de passes par ce même facteur pour avoir au final la même dose de traitement exprimée en ion/cm². Dans le cas d'un défilement en continu, on peut multiplier selon le même rapport le nombre de micro accélérateurs placés par exemple sur le parcours d'une bande.

On constate en outre que d'autres propriétés de surface sont très significativement améliorées grâce à un traitement selon l'invention et que des performances qui ne semblent pas pouvoir être atteintes avec d'autres techniques ont été mises en évidence.

L'invention ne se limite pas à ces types de réalisation et doit être interprétée de façon non limitative, et englobant le traitement de tout type de polymère.

De même le procédé selon l'invention n'est pas limité à l'utilisation d'une source RCE, et même si on peut penser que d'autres sources seraient moins avantageuses, on peut mettre en oeuvre le procédé selon l'invention avec des sources mono-ions ou d'autres sources multi-ions, du moment que ces sources sont configurées de manière à permettre une implantation simultanée d'ions multi-énergies appartenant à liste constituée de l'hélium (He), l'azote,(N) l'oxygène (O), le néon (Ne), l'argon (Ar), le krypton (Kr), le xénon (Xe).

Diverses modifications sont également possibles pour un homme du métier sans sortir du cadre de la présente invention tel que défini par les revendications annexées.

## Revendications

1. Procédé de traitement de surface élastomère d'un dispositif de distribution de produit fluide, **caractérisé en ce que** ledit procédé comprend l'étape de modifier par implantation ionique, au moyen de faisceaux d'ions multichargés et multi-énergies, au moins une surface élastomère à traiter dudit dispositif, de telle sorte que ladite surface élastomère modifiée limite le collage des surfaces élastomères lors des phases de fabrication et/ou d'assemblage, l'implantation ionique étant réalisée à une profondeur de 0 à 3 µm, ledit faisceau d'ion étant constitué d'ions multi-énergies X+ et X2+, où X est le symbole atomique de l'ion choisi parmi la liste comprenant l'hélium (He), l'azote (N), l'oxygène (O), le néon (Ne), l'argon (Ar), le krypton (Kr), le xénon (Xe), dans lequel RX = X+/X2+ avec X+ et X2+ exprimés en pourcentage atomique est inférieur ou égal à 100, par exemple inférieur à 20, dans lequel la vitesse de déplacement du faisceau est égale à la plus petite vitesse de déplacement du faisceau qui ne provoque pas une dégradation thermique du polymère se manifestant par une remontée de pression de 10-5 mbar, telle qu'identifiée dans une étape préalable.

2. Procédé selon la revendication 1, dans lequel lesdits ions multi-énergies sont implantés simultanément avec la même tension d'extraction.

3. Procédé selon l'une quelconque des revendications précédentes, dans lequel des ions d'hélium multi-énergies He+ et He2+ sont implantés simultanément.

4. Procédé selon la revendication 3, dans lequel le ratio RHe, où RHe = He+/He2+ avec He+ et He2+ exprimés en pourcentage atomique, est inférieur ou égal à 100, par exemple inférieur à 20, et supérieur à 1.

5. Procédé selon l'une quelconque des revendications précédentes, dans lequel la température de la surface élastomère en cours de traitement est inférieure à 100°C, de préférence inférieure à 50°C.

6. Procédé selon l'une quelconque des revendications précédentes, dans lequel les ions X+ et X2+ sont produits simultanément par une source d'ions à résonnance cyclotronique électronique (RCE).

7. Procédé selon l'une quelconque des revendications précédentes, dans lequel le ratio RX est supérieur ou égal à 1.

8. Procédé selon l'une quelconque des revendications précédentes, dans lequel la tension d'extraction de la source permettant l'implantation des ions multi-énergies X+ et X2+ est comprise entre 10 et 400 kV, par exemple supérieure ou égale à 20 kV et/ou inférieure ou égale à 100 kV.

9. Procédé selon l'une quelconque des revendications précédentes, dans lequel la dose d'ion multi-énergie X+ et X2+ est comprise entre 5.1014 et 1018 ions/cm2, par exemple supérieure ou égale à 1015 ions/cm2 et/ou inférieure ou égale à 5.1017 ions/cm2, voire supérieure ou égale à 5.1015 ions/cm2 et/ou inférieure ou égale à 1017 ions/cm2.

10. Procédé selon l'une quelconque des revendications précédentes, dans lequel on détermine dans une étape préalable la variation d'une propriété caractéristique de l'évolution de la surface d'une pièce massive en polymère, par exemple la résistivité électrique superficielle de la surface, ρ, d'un matériau polymère représentatif de celui de la pièce à traiter en fonction de doses d'ions multi-énergie X+ et X2+ de manière à déterminer un domaine de doses d'ions où la variation de la propriété caractéristique choisie est avantageuse et évolue de manière différenciée dans trois zones consécutives de doses d'ions formant ledit domaine de doses d'ions, avec une évolution dans la première zone sensiblement linéaire et réversible sur une durée inférieure à un mois, une évolution dans la deuxième zone sensiblement linéaire et stable sur une durée supérieure à un mois enfin une évolution dans la troisième zone constante et stable sur une durée supérieure à un mois et où l'on choisit la dose d'ions multi énergie X+ et X2+ dans la troisième zone de doses d'ions pour traiter la pièce massive en polymère.

11. Procédé selon l'une quelconque des revendications précédentes, dans lequel on règle des paramètres de la source et de déplacement de la surface de la pièce en polymère à traiter de manière à ce que la vitesse surfacique de traitement de la surface de la pièce en polymère à traiter est comprise entre 0,5 cm2/s et 1000 cm2/s, par exemple supérieure ou égale à 1 cm2/s et/ou inférieure ou égale à 100 cm2/s.

12. Procédé selon l'une quelconque des revendications précédentes, dans lequel on règle des paramètres de la source et de déplacement de la surface de la pièce en polymère à traiter de manière à ce que la dose d'ion implantée soit comprise entre 5.1014 et 1018 ions/cm2, par exemple supérieure ou égale à 5.1015 ions/cm2 et/ou inférieure ou égale à 1017 ions/cm2.

13. Procédé selon l'une quelconque des revendications précédentes, dans lequel on règle des paramètres de la source et de déplacement de la surface de la pièce en polymère à traiter de manière à ce que la profondeur de pénétration de l'ion sur la surface de la pièce en polymère traitée soit comprise entre 0,05 et 3 µm, par exemple supérieure ou égale à 0,1 µm et/ou inférieure ou égale à 2 µm.

14. Procédé selon l'une quelconque des revendications précédentes, dans lequel on règle des paramètres de la source et de déplacement de la surface de la pièce en polymère à traiter de manière à ce que la température de la surface de la pièce en polymère en cours de traitement soit inférieure ou égale à 100°C, par exemple inférieure ou égale à 50°C.

15. Procédé selon l'une quelconque des revendications précédentes, dans lequel la pièce en polymère à traiter défile dans un dispositif de traitement, par exemple à une vitesse comprise entre 5 m/min et 100 m/min.

16. Procédé selon l'une quelconque des revendications précédentes, dans lequel l'implantation d'ion de la surface en polymère de la pièce à traiter est effectuée grâce à une pluralité de faisceaux d'ions multi énergies X+ et X2+ produits par une pluralité de sources d'ions.

17. Procédé selon l'une quelconque des revendications précédentes, dans lequel ledit dispositif de distribution comporte un réservoir contenant le produit fluide, un organe de distribution, tel qu'une pompe ou une valve, fixé sur ledit réservoir, et une tête de distribution pourvue d'un orifice de distribution, pour actionner ledit organe de distribution.

18. Procédé selon l'une quelconque des revendications précédentes, dans lequel ladite surface élastomère est un joint de col et/ou un joint de soupape d'un organe de distribution, tel qu'une pompe ou une valve.

19. Procédé selon l'une quelconque des revendications précédentes, dans lequel ledit produit fluide est un produit fluide pharmaceutique destiné à être pulvérisé et/ou inhalé de manière nasale ou orale.

20. Procédé selon l'une quelconque des revendications précédentes, dans lequel ledit procédé est réalisé en continu sur la chaîne de montage et d'assemblage du dispositif de distribution de produit fluide.

## Patentansprüche

1. Verfahren zur Behandlung einer elastomeren Oberfläche einer Ausgabevorrichtung für ein fluides Produkt, **dadurch gekennzeichnet, dass** das Verfahren den Schritt des Modifizierens mindestens einer zu behandelnden elastomeren Oberfläche der Vorrichtung durch Ionenimplantation mittels mehrfach geladener Multienergie-Ionenstrahlen umfasst, so dass die modifizierte elastomere Oberfläche das Kleben der elastomeren Oberflächen während der Herstellungs- und/oder Montagephasen begrenzt, wobei die Ionenimplantation in einer Tiefe von 0 bis 3 µm vorgenommen wird, wobei der Ionenstrahl aus Multienergie-Ionen X+ und X2+ besteht, wobei X das Elementsymbol des Ions ist, ausgewählt aus der Liste aufweisend Helium (He), Stickstoff (N), Sauerstoff (O), Neon (Ne), Argon (Ar), Krypton (Kr), Xenon (Xe), wobei RX = X+/X2+, wobei X+ und X2+ in Atomprozent ausgedrückt sind, kleiner oder gleich 100, zum Beispiel kleiner als 20 ist, wobei die Verschiebungsgeschwindigkeit des Strahls gleich der kleinsten Verschiebungsgeschwindigkeit des Strahls ist, die keine thermische Beeinträchtigung des Polymers hervorruft, die sich durch einen Druckanstieg von 10-5 mbar zeigt, wie in einem vorausgehenden Schritt identifiziert wird.

2. Verfahren nach Anspruch 1, wobei die Multienergie-Ionen gleichzeitig mit der gleichen Extraktionsspannung implantiert werden.

3. Verfahren nach einem der vorhergehenden Ansprüche, wobei Multienergie-Heliumionen He+ und He2+ gleichzeitig implantiert werden.

4. Verfahren nach Anspruch 3, wobei das Verhältnis RHe, wobei RHe = He+/He2+ ist, wobei He+ und He2+ in Atomprozent ausgedrückt sind, kleiner oder gleich 100 ist, zum Beispiel kleiner als 20 und größer als 1.

5. Verfahren nach einem der vorhergehenden Ansprüche, wobei die Temperatur der elastomeren Oberfläche bei der Behandlung weniger als 100 °C, vorzugsweise weniger als 50 °C beträgt.

6. Verfahren nach einem der vorhergehenden Ansprüche, wobei die Ionen X+ und X2+ gleichzeitig von einer Elektron-Zyklotron-Resonanz-Ionenquelle (EZR) erzeugt werden.

7. Verfahren nach einem der vorhergehenden Ansprüche, wobei das Verhältnis RX größer oder gleich 1 ist.

8. Verfahren nach einem der vorhergehenden Ansprüche, wobei die Extraktionsspannung der Quelle, die die Implantation der Multienergie-Ionen X+ und X2+ ermöglicht, zwischen 10 und 400 kV beträgt, zum Beispiel größer oder gleich 20 kV und/oder kleiner oder gleich 100 kV.

9. Verfahren nach einem der vorhergehenden Ansprüche, wobei die Multienergie-Ionendosis X+ und X2+ zwischen 5.1014 und 1018 Ionen/cm2, zum Beispiel größer oder gleich 1015 Ionen/cm2 und/oder kleiner oder gleich 5.1017 Ionen/cm2 bzw. größer oder gleich 5.1015 Ionen/cm2 und/oder kleiner oder gleich 1017 Ionen/cm2 liegt.

10. Verfahren nach einem der vorhergehenden Ansprüche, wobei in einem vorausgehenden Schritt die Abweichung einer charakteristischen Eigenschaft der Entwicklung der Oberfläche eines massiven Stücks aus Polymer, zum Beispiel des spezifischen elektrischen Oberflächenwiderstands, p, eines polymeren Materials, bestimmt wird, das für dasjenige des zu behandelnden Stücks repräsentativ ist, und zwar in Abhängigkeit der Dosen der Multienergie-Ionen X+ und X2+, um einen Bereich von Ionendosen zu bestimmen, in dem die Abweichung der gewählten charakteristischen Eigenschaft vorteilhaft ist und sich in drei aufeinanderfolgenden Zonen von Ionendosen, die den Ionendosenbereich bilden, unterschiedlich entwickelt, mit einer im Wesentlichen linearen und umkehrbaren Entwicklung in der ersten Zone über eine Dauer von weniger als einem Monat, einer im Wesentlichen linearen und stabilen Entwicklung in der zweiten Zone über eine Dauer von mehr als einem Monat, dann einer konstanten und stabilen Entwicklung in der dritten Zone über eine Dauer von mehr als einem Monat, wobei die Dosis der Multienergie-Ionen X+ und X2+ in der dritten Zone aus Ionendosen zur Behandlung des massiven Stücks aus Polymer gewählt wird.

11. Verfahren nach einem der vorhergehenden Ansprüche, wobei Parameter der Quelle und der Verschiebung der Oberfläche des zu behandelnden Stücks aus Polymer so eingestellt werden, dass die flächenbezogene Behandlungsgeschwindigkeit der Oberfläche des zu behandelnden Stücks aus Polymer zwischen 0,5 cm2/s und 1000 cm2/s liegt, zum Beispiel größer oder gleich 1 cm2/s und/oder kleiner oder gleich 100 cm2/s beträgt.

12. Verfahren nach einem der vorhergehenden Ansprüche, wobei Parameter der Quelle und der Verschiebung der Oberfläche des zu behandelnden Stücks aus Polymer so eingestellt werden, dass die implantierte Ionendosis zwischen 5.1014 und 1018 Ionen/cm2 beträgt, zum Beispiel größer oder gleich 5.1015 Ionen/cm2 und/oder kleiner oder gleich 1017 Ionen/cm2.

13. Verfahren nach einem der vorhergehenden Ansprüche, wobei Parameter der Quelle und der Verschiebung der Oberfläche des zu behandelnden Stücks aus Polymer so eingestellt werden, dass die Eindringungstiefe des Ions auf der Oberfläche des zu behandelnden Stücks aus Polymer zwischen 0,05 und 3 µm liegt, zum Beispiel größer oder gleich 0,1 µm und/oder kleiner oder gleich 2 µm.

14. Verfahren nach einem der vorhergehenden Ansprüche, wobei Parameter der Quelle und der Verschiebung der Oberfläche des zu behandelnden Stücks aus Polymer so eingestellt werden, dass die Temperatur der Oberfläche des Stücks aus Polymer während der Behandlung kleiner oder gleich 100 °C, zum Beispiel kleiner oder gleich 50 °C beträgt.

15. Verfahren nach einem der vorhergehenden Ansprüche, wobei das zu behandelnde Stück aus Polymer in eine Behandlungsvorrichtung einläuft, zum Beispiel mit einer Geschwindigkeit zwischen 5 m/min und 100 m/min.

16. Verfahren nach einem der vorhergehenden Ansprüche, wobei die Ionenimplantation der Oberfläche aus Polymer des zu behandelnden Stücks durch mehrere Multienergie-Ionenstrahlen X+ und X2+ erfolgt, die durch mehrere Ionenquellen erzeugt werden.

17. Verfahren nach einem der vorhergehenden Ansprüche, wobei die Ausgabevorrichtung einen Behälter, der das fluide Produkt enthält, eine auf dem Behälter befestigte Ausgabeeinrichtung, wie eine Pumpe oder ein Ventil, und einen mit einer Ausgabeöffnung versehenen Ausgabekopf, um die Ausgabeeinrichtung zu betätigen, aufweist.

18. Verfahren nach einem der vorhergehenden Patentansprüche, wobei die elastomere Oberfläche eine Kragendichtung und/oder eine Ventildichtung einer Ausgabeeinrichtung, wie eine Pumpe oder ein Ventil, ist.

19. Verfahren nach einem der vorhergehenden Ansprüche, wobei das fluide Produkt ein pharmazeutisches fluides Produkt ist, das zum Pulverisieren und/oder Inhalieren durch die Nase oder den Mund gedacht ist.

20. Verfahren nach einem der vorhergehenden Ansprüche, wobei das Verfahren kontinuierlich auf dem Montageband der Ausgabevorrichtung für ein fluides Produkt erfolgt.

## Claims

1. A method of treating an elastomer surface of a fluid dispenser device, **characterized in that** said method comprises a step of modifying at least one elastomer surface to be treated of said device by ionic implantation using multi-charged and multi-energy ion beams, such that said modified elastomer surface limits adhesion of the elastomer surfaces during the manufacturing and/or assembly stages, ionic implantation being carried out to a depth of 0 µm to 3 µm, said ion beams being constituted by multi-energy ions X⁺ and X²⁺, where X is the atomic symbol of the ion selected from the list comprising helium (He), nitrogen (N), oxygen (O), neon (Ne), argon (Ar), krypton (Kr), and xenon (Xe), wherein RX = X⁺/X²⁺, with X⁺ and X²⁺, expressed as atomic percentages, being less than or equal to 100, for example less than 20, and wherein the movement speed of the beam is equal to the lowest movement speed of the beam that does not cause thermal degradation of the polymer, as manifested by an increase in pressure of 10⁻⁵ mbar, as identified in a prior step.

2. A method according to claim 1, wherein said multi-energy ions are implanted simultaneously with the same extraction voltage.

3. A method according to either preceding claim, wherein multi-energy helium ions He⁺ and He²⁺ are implanted simultaneously.

4. A method according to claim 3, wherein the ratio RHe, where RHe = He⁺/He²⁺, where He⁺ and He²⁺ are expressed as atomic percentages, is less than or equal to 100, for example less than 20, and more than 1.

5. A method according to any preceding claim, wherein the temperature of the elastomer surface during treatment is less than 100°C, preferably less than 50°C.

6. A method according to any preceding claim, wherein the ions X⁺ and X²⁺ are produced simultaneously by an electron cyclotron resonance ion source (ECR).

7. A method according to any preceding claim, wherein the ratio RX is greater than or equal to 1.

8. A method according to any preceding claim, wherein the extraction voltage of the source allowing implantation of multi-energy ions X⁺ and X²⁺ is in the range 10 kV to 400 kV, for example greater than or equal to 20 kV and/or less than or equal to 100 kV.

9. A method according to any preceding claim, wherein the dose of multi-energy ions X⁺ and X²⁺ is in the range 5×10¹⁴ ions/cm² to 10¹⁸ ions/cm², for example greater than or equal to 10¹⁵ ions/cm² and/or less than or equal to 5×10¹⁷ ions/cm² or even greater than or equal to 5×10¹⁵ ions/cm² and/or less than or equal to 10¹⁷ ions/cm².

10. A method according to any preceding claim, wherein in a previous step, the variation as a function of the dose of multi-energy ions X⁺ and X²⁺ in a characteristic property of the change of the surface of a solid polymer part, for example the electrical resistivity of the surface, ρ, of a polymer material representative of that of the part to be treated, is determined in order to determine a range of ion doses wherein the variation in the selected characteristic property is advantageous and varies in different ways in three consecutive zones of ion doses forming said ion doses range, with a change in the first zone that is substantially linear and reversible over a period of less than one month, a change in the second zone that is substantially linear and stable over a period of more than one month, and finally a change in the third zone that is constant and stable over a period of more than one month, and wherein the dose of multi-energy ions X⁺ and X²⁺ in the third ion dose zone is selected to treat the solid polymer part.

11. A method according to any preceding claim, wherein the parameters of the source and of the movement of the surface of the polymer part to be treated are adjusted such that the areal speed of the surface of the polymer part to be treated is in the range 0.5 cm²/s to 1000 cm²/s, for example greater than or equal to 1 cm²/s and/or less than or equal to 100 cm²/s.

12. A method according to any preceding claim, wherein the parameters of the source and of the movement of the surface of the polymer part to be treated are adjusted such that the implanted ion dose is in the range 5×10¹⁴ ions/cm² to 10¹⁸ ions/cm², for example greater than or equal to 5×10¹⁵ ions/cm² and/or less than or equal to 10¹⁷ ions/cm².

13. A method according to any preceding claim, wherein the parameters of the source and of the movement of the surface of the polymer part to be treated are adjusted such that the penetration depth of the ion on the surface of the treated polymer part is in the range 0.05 µm to 3 µm, for example greater than or equal to 0.1 µm and/or less than or equal to 2 µm.

14. A method according to any preceding claim, wherein the parameters of the source and of the movement of the surface of the polymer part to be treated are adjusted such that the temperature of the surface of the polymer part during treatment is less than or equal to 100°C, for example less than or equal to 50°C.

15. A method according to any preceding claim, wherein the polymer part to be treated runs past a treatment device, for example at a speed in the range 5 m/min to 100 m/min.

16. A method according to any preceding claim, wherein ion implantation from the surface of the polymer part to be treated is carried out by means of a plurality of multi-energy beams of X⁺ and X²⁺ ions produced by a plurality of ion sources.

17. A method according to any preceding claim, wherein said dispenser device comprises a reservoir containing the fluid, a dispenser member such as a pump or a valve attached to said reservoir, and a dispenser head provided with a dispenser orifice in order to actuate said dispenser member.

18. A method according to any preceding claim, wherein said elastomer surface is a neck gasket and/or a valve gasket of a dispenser member, such as a pump or a valve.

19. A method according to any preceding claim, wherein said fluid is a pharmaceutical fluid for spraying and/or inhaling nasally or orally.

20. A method according to any preceding claim, wherein said method is carried out continuously on an assembly line for the fluid dispenser device.
